# EUROPÄISCHE PATENTSCHRIFT

(11) **EP 3 352 709 B1**
(45) Veröffentlichungstag und Bekanntmachung des Hinweises auf die Patenterteilung: **29.09.2021**
(21) Anmeldenummer: 16753246.4
(22) Anmeldetag: 04.08.2016
(51) Int. Cl.: A61F 2/30, A61F 2/44, A61F 2/46, A61B 17/02

(54) **VORRICHTUNG FÜR WIRBELSÄULENEINGRIFFE, ZUGEHÖRIGE FÜHRUNGSHÜLSE UND SET MIT FÜHRUNGSHÜLSE**
DEVICE FOR SPINAL COLUMN INTERVENTIONS, ASSOCIATED GUIDE SLEEVE, AND KIT WITH GUIDE SLEEVE
DISPOSITIF POUR DES INTERVENTIONS SUR LA COLONNE VERTÉBRALE, DOUILLE DE GUIDAGE ASSOCIÉE ET SET COMPRENANT LA DOUILLE DE GUIDAGE

(30) Priorität: 23.09.2015 DE 102015012171
(43) Veröffentlichungstag der Anmeldung: 01.08.2018
(73) Patentinhaber: Joimax GmbH, 76227 Karlsruhe (DE)
(72) Erfinder: RIES, Wolfgang, 76351 Linkenheim-Hochstetten (DE)
(74) Vertreter: Lichti - Patentanwälte Partnerschaft mbB
(86) Internationale Anmeldenummer: PCT/EP2016/001342
(87) Internationale Veröffentlichungsnummer: WO 2017/050410

(56) Entgegenhaltungen:
- DE-U1-202013 007 361
- DE-U1-202014 003 441
- US-A1- 2005 119 747
- US-A1- 2006 247 654
- US-A1- 2007 073 399
- US-A1- 2008 255 563
- US-A1- 2015 250 612
- US-B1- 6 395 031

## Beschreibung

Die Erfindung betrifft eine Vorrichtung für Wirbelsäuleneingriffe nach dem Oberbegriff des Anspruchs 1.

In der Wirbelsäulenchirurgie werden mehr und mehr herkömmliche Eingriffe, bei denen durch erhebliche Schnitte im Rücken des Patienten und Durchtrennen von Muskeln die Wirbelsäule im zu operierenden Bereich freigelegt wird, durch perkutane Wirbelsäulen-Eingriffe ersetzt. Um Wege zum Einführen von Instrumenten durch Haut und der Wirbelsäule bzw. den Wirbeln vorgelagertes Gewebe sowie ein perkutanes Arbeiten mit solchen Geräten zu ermöglichen, wird eine Führungs- oder Arbeitshülse mit hinreichendem Lumen und damit Querschnitt eingeführt. Diese kann nicht ohne weiteres als solche eingeführt werden. Es erfolgt vielmehr zunächst ein Durchstoßen von Haut und darunterliegendem Gewebe mit einem dünnen Nadelset aus einem Stilett und einer dieses eng umgebenden Hohlnadel, die sich beide distal verjüngen, also zugespitzt sind (Jamshidi-Nadel). Anschließend wird das Stilett aus der Hohlnadel entfernt, durch diese ein Führungsdraht bis zum Bandscheibenfach oder Zwischenwirbelfach geführt. Dann wird die Hohlnadel entfernt und über den Führungsdraht wird ein erster Dilator eingeführt. Über diesen werden jeweils weitere Dilatoren eingeführt, die mit ihrem Lumen an die Außenkontur des vorher eingeführten Dilators eng angepasst sind. Die Dilatoren sind an ihrem Ende konisch verjüngt. Dies erfolgt so lange, bis über einen letzten Dilator dann die Führungs- oder Arbeitshülse eingeführt werden kann, aus der dann Führungsdraht und Dilatoren entfernt werden und durch dessen Lumen dann das Arbeiten des Chirurgen durch Einführen von Instrumenten etc. und über diese oder durch Einführen eines Zwischenwirbel-Körbchens (Interbody Cage) in das Zwischenwirbelfach erfolgen kann. Sämtliche vorgenannten und auch die weiteren Schritte erfolgen unter Röntgenbeobachtung.

Die Dilatoren und insbesondere die Arbeits- oder Führungshülse sind bisher regelmäßig zylindrisch ausgebildet. Es hat sich herausgestellt, dass insbesondere beim Einführen eines Zwischenwirbel-Körbchens die alleine durch das perkutane Vorgehen gegenüber einer offenen Operation wesentlich reduzierte Belastung des Patienten doch noch unerwünscht ist, da insbesondere die seitlichen Querschnittsabmessungen eines Körbchens recht groß sind und daher der Durchmesser einer herkömmlichen zylindrischen Führungshülse ebenfalls recht groß sein muss.

Die US 2007/0073399 A1 zeigt eine Vorrichtung zum Manipulieren und Revitalisieren einer Bandscheibe mittels eines minimalinvasiven Eingriffs. Das Einführinstrumentarium weist hierzu einen Führungsdraht auf, der durch Inzision mittels einer durch einen Obturator (Stilett) verschlossenen Hohlnadel geschaffenen Einstichkanal nach Entfernen des Obturators durch die Hohlnadel eingeführt werden kann, woraufhin diese entfernt wird und über den Führungsdraht weitere Instrumente geführt werden, so der weiterhin zu dem Apparat der Druckschrift gehörenden Dilator, über den nach Aufweiten des Einstichkanals eine Kanüle bis zum Eingriffsort eingeschoben werden kann. Nach Entfernen des Dilators als weiteren Instrumententeil des Systems kann ein "driver" eingeschoben werden und dann der intendierte Eingriff am Operationsort vorgenommen werden kann, wie eben die Manipulation oder Revitalisierung einer Bandscheibe oder eines Teils derselben.

Insbesondere der Dilator weist im Schnitt eine rechteckige Außenkontur auf und demgemäß die Kanüle eine rechteckige Innenkontur, die wiederum eine rechteckige Außenkontur aufweist. Die rechteckige Innenkontur begrenzt, soweit Instrumente diese ausfüllen sollen, deren Querschnitt, anderenfalls bedingt sie eine größere unnötige Aufweitung des Kanals im Gewebe eines Patienten als dies durch ein konkretes Instrument notwendig wäre. Darüber hinaus hat die rechteckige Kanüle scharfe Außenkanten. Dies ist nachteilig, da sie durch das Weichgewebe zum einen an Blutgefäßen vorbeigeführt werden muss, zum anderen im Einführungsbereich auch Nervenbahnen liegen, die beide durch die scharfen Kanten beschädigt werden können.

Die US 6,395,031 B1 sieht die zur Reparatur beschädigter Wirbelscheiben seitlich expandierende Abstandshalter vor. Die Abstandshalter sollen die Höhe eines Bandscheibenraums aufrechterhalten und gleichzeitig Stabilität für die Wirbelsäule bieten. Der Wirbelabstandshalter weist ein Paar Seitenarme auf, die sich zwischen einem distalen Ende und einem proximalen Ende erstrecken. Der Halter hat eine obere Wirbellagerfläche und eine identische untere Wirbellagerfläche. Ein zentraler Hohlraum ist zwischen den Seitenarmen ausgebildet. Der Halter hat auch Öffnungen, die durch die Seitenarme definiert sind. Die Öffnungen ermöglichen eine Verbindung zwischen dem Inneren und dem Äußeren des Halters und reduzieren das Material in den Wänden, wodurch die Flexibilität des Halters erhöht wird. Der Halter ist vorzugsweise aus einem elastisch flexiblen Material gebildet. Der Halter kann durch ein röhrenförmiges Abgabesystem wie eine Kanüle eingeführt werden. Diese ist im Querschnitt quadratisch mit zeichnerisch abgerundeten Kanten ausgebildet, denen in der Druckschrift keine besondere Bedeutung zugeschrieben wird. Sobald der Halter in den Zwischenwirbel eingeführt ist, wird er nicht länger durch die Kanüle begrenzt und dehnt sich seitlich zu einer zweiten Konfiguration innerhalb des Zwischenwirbelraums aus, die sich ihrem Zustand vor dem Einsetzen annähert.

Die DE 20 2013 007 361 U1 zeigt ein Instrumentenset zum Einbringen eines Körbchens in das Bandscheibenfach zwischen zwei Wirbelkörpern mit einem Führungsdraht, mit mehreren über den Führungsdraht und übereinander aufschiebbaren Dilatoren, mit einer Arbeitshülse und einem Körbchen, wobei die grundsätzlich zylindrische, also im Querschnitt kreisförmige Arbeitshülse in ihrem distalen Bereich derart ausgebildet ist, dass sie ein Festlegen ihres distalen Bereichs in Richtung ihrer Erstreckung bei angularer Beweglichkeit oder variabler angularer Ausrichtbarkeit ihres proximalen Endes ermöglicht. Die Druckschrift zeigt dabei nicht nur ein Zwischenwirbelimplantat in Form eines Körbchens oder Käfigs mit an den Zwischenwirbelraum angepasster Kontur, sondern auch ein gattungsgemäßes Einführbesteck mit mehreren Dilatoren und einer Führungshülse, die zylindrisch ausgebildet ist, also kreisförmigen Querschnitt hat.

Die US 2006/247654 A1 zeigt ein nicht gattungsgemäßes Fräsinstrument zur Vorbereitung einer Wirbelendplatte entlang eines Schneidwegs, der schräg zur Annäherungsachse an den Wirbel ausgerichtet ist. Das Fräsinstrument ermöglicht die Steuerung der anterior-posterioren Tiefe der Endplattenentfernung durch Führen der Schneidanordnung im Scheibenraum, wenn sie sich quer über eine Gehäuseanordnung bewegt, zeigt sie ein Führungsrohr mit einem quadratischen Querschnitt entsprechend den Druckschriften US 2007/0073399 A1 und US 6,395,031 B1, wobei das Führungsrohr ebenso wie dasjenige der Druckschrift D8 an seinen Kanten abgerundet ist. Darüber hinaus werden ohne Darstellung und nähere Qualifizierung allgemein Führungsrohre für eben Fräsinstrumente mit kreisförmigem, ovalem, acht- oder ganz pauschal anderen Querschnittskonfigurationen erwähnt.

Der Erfindung liegt daher die Aufgabe zugrunde, eine Vorrichtung und eine Führungshülse für Wirbelsäuleneingriffe, sowie ein Set aus Führungshülse und Zwischenwirbel-Körbchen zu schaffen, bei deren Einsatz die Belastung des Patienten bei einem perkutanen Wirbelsäuleneingriff weiter wesentlich reduziert werden kann.

Erfindungsgemäß wird die genannte Aufgabe mit einer Vorrichtung für einen Wirbelsäuleneingriff der eingangs genannten Art gelöst, die die kennzeichnenden Merkmale des Anspruchs 1 aufweist.

Die erfindungsgemäße Vorrichtung, die auch als Einführset benutzt wird, weist demgemäß mindestens eine in der vorgenannten Form ausgebildeten Führungshülse sowie mindestens einen - ersten oder äußersten - Dilator (der aber dennoch innerhalb der Führungshülse angeordnet ist bzw. über den die Führungshülse eingeführt wird) auf, dessen Außenkontor an das Innenlumen der Führungshülse angepasst ist.

Durch die erfindungsgemäße Ausgestaltung der Vorrichtung und der Führungshülse selbst sowie des eine solche beinhaltenden Implantations-Sets wird erreicht, dass die Belastung eines Patienten, bei dem durch eine solche Führungshülse hin ein perkutaner Eingriff, insbesondere das Einbringen eines Zwischenwirbel-Körbchens erfolgt, wesentlich reduziert wird, da die Höhe der Führungshülse gegenüber herkömmlichen zylindrischen Führungshülsen deutlich reduziert ist, so dass in Richtung der Höhe (also in Erstreckungsrichtung der Wirbelsäule) eine deutlich geringere Aufweitung und damit eben eine geringere Kompressionsgefahr für die austretende Nervenwurzel gegeben ist. Die Vorrichtung ist insbesondere vorteilhaft bei einer geringen Höhe des Bandscheibenfachs eines Patienten.

In einer bevorzugten Weiterbildung sieht die Erfindung einen Krümmungsradius von 11 bis 12,5 mm der konvex gekrümmten Breitseite vor. Darüber hinaus sieht die Erfindung in bevorzugter Ausgestaltung vor, dass der Krümmungswinkel des Übergangs zwischen einer Breitseite und einer Schmalseite zwischen 3 und 4,5 mm liegt.

Gemäß einer äußerst bevorzugten Weiterbildung der Erfindung ist vorgesehen, dass die Schmalseiten der Führungshülse distal die Breitseiten überragende Vorsprünge aufweisen, wobei insbesondere die Vorsprünge sich zu ihrem freien Ende hin verjüngen und am freien Ende abgerundet sind und/oder die Schmalseiten eine größere Stärke als die Breitseiten aufweisen, vorzugsweise die Stärke der Breitseiten 0,8 mm bis 1,2 mm und/oder die Stärke der Schmalseiten größer 1,2 mm bis 1,7 mm beträgt.

Das aus Führungshülse und unmittelbar an diese in seiner Außenkontur angepassten Dilator bestehende Einführset sieht in Weiterbildung insbesondere vor, dass das Lumen des Dilators zylindrisch ausgebildet ist. Hierdurch wird erreicht, dass die weiteren inneren Dilatoren, die zuerst über den gelegten Führungsdraht bzw. übereinander durch Haut und Gewebe zur Eingriffsstelle an der Wirbelsäule eingeführt werden weiterhin in bekannter Weise mit zylindrischer Kontur ausgebildet sein können, ohne dass dies zu einer erhöhten Belastung des Patienten führt bzw. die durch die erfindungsgemäße Ausgestaltung von Führungshülse und letztem Dilator, über den die Führungshülse unmittelbar eingeführt wird, erreichte geringere Belastung beeinträchtigt wird.

In weiter bevorzugter Ausgestaltung ist vorgesehen, dass Hülse und/oder Dilator quer zu ihrer Längsachse verlaufende Nuten im proximalen Bereich ihrer Außenseite aufweisen, insbesondere an ihren Schmalseiten. Diese Nuten dienen zur Gewährleistung einer besseren Griffigkeit für Finger oder ein Werkzeug zum Herausziehen von Hülse bzw. Dilator. Eine Weiterbildung sieht vor, dass im proximalen Zahn/Keilbereich diametral gegenüberliegende Durchbrüche vorhanden sind.

Schließlich ist bei dem Implantations-Set zum Wirbelsäuleneingriff aus Führungshülse und Zwischenwirbel-Körbchen vorgesehen, dass die Führungshülse in bevorzugt entsprechend einer oder mehreren der vorstehend beschriebenen Ausgestaltungen ausgebildet ist.

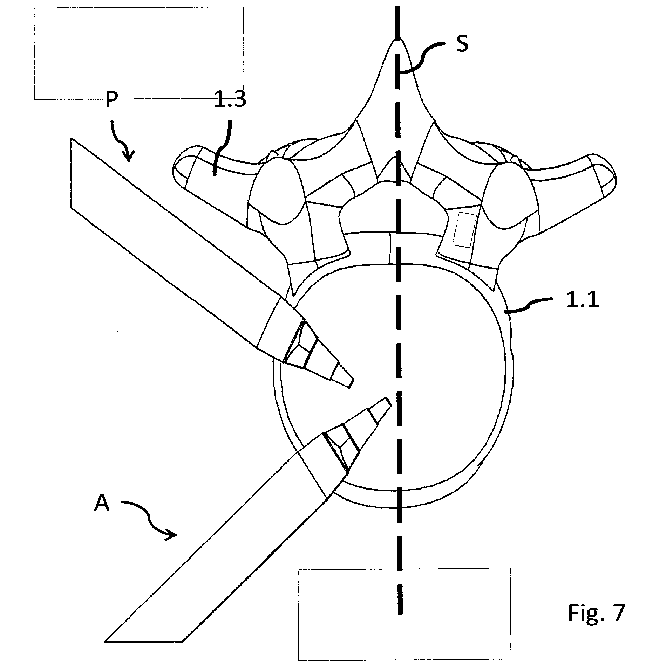

Der Zugang zum Zwischenraum (Bandscheibenfach) zwischen zwei Wirbeln mittels der erfindungsgemäßen Vorrichtung ist sowohl posterior-lateral als auch anterior-lateral möglich und zwar vorzugsweise mit einem Zugangswinkel von jeweils 30° bis 60°, vorzugsweise 40° bis 50° jeweils zur - mittleren - Sagittalebene bzw. in diese liegende - horizontale - Sagittalachse jeweils oben von seitlich hinten bzw. vorne, wie dies in Fig. 7 dargestellt ist. Der posterior-laterale Zugang kann dabei vorzugsweise entlang der äußeren Seitenfläche des Dornfortsatzes des Wirbels erfolgen.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im Einzelnen erläutert ist. Dabei zeigt:
- Fig. 1.1: eine Seitenansicht-Darstellung einer Führungshülse der erfindungsgemäßen Vorrichtung mit zwischen zwei Wirbeln ins Bandscheibenfach ragenden distalen Vorsprüngen;
- Fig. 1.2: eine Stirnseite der Führungshülse der Fig. 1 mit eingeführtem Zwischenwirbel-Körbchen (Interbody Cage);
- Fig. 1.3: eine perspektivische Sicht der Ausgestaltung der Fig. 1 mit am proximalen Ende in die Führungshülse eingeführten Zwischenwirbelkörbchen;
- Fig. 2: eine schematische Darstellung eines typischen Zwischenwirbel-Körbchens in perspektivischer Sicht;
- Fig. 3: eine Draufsicht auf das proximale Ende einer Führungshülse mit eingeführtem Körbchen;
- Fig. 4.1: eine perspektivische Darstellung einer Führungshülse, schräg von ihrem distalen Ende her gesehen;
- Fig. 4.2: eine Seitenansicht der Führungshülse der Fig. 6.1;
- Fig. 5.1: eine perspektivische Darstellung eines unmittelbar innerhalb der Führungshülse zum Einsatz kommenden nichtzylindrischen Dilators einer erfindungsgemäßen Vorrichtung;
- Fig. 5.2: eine Seitenansicht des Dilators der Fig. 5.1;
- Fig. 6: Darstellungen der erfindungsgemäßen Vorrichtung mit einer Führungshülse im Satz mit zwei in diese eingeführten Dilatoren, dabei zeigt
- Fig. 6.1: eine Draufsicht von oben;
- Fig. 6.2: eine Seitenansicht;
- Fig. 6.3: eine perspektivische Darstellung vom proximalen Ende her;
- Fig. 6.4: eine perspektivische Darstellung zum distalen Ende;
- Fig. 6.5: eine vergrößerte Darstellung des distalen Endes des Satzes der Fig. 6.1 bis 6.4; und
- Fig. 7: eine Darstellung zum alternativen posteriorlateralen oder anterior-lateralen Zugang zum Bandscheibenfach.

Bei Bandscheibenschäden, bei denen diese teilweise und insbesondere vollständig entfernt werden müssen, ist zur Stabilisierung der Wirbelsäule zwischen die der betroffenen Bandscheibe benachbarten Wirbel 1.1, 1.2 mindestens ein Zwischenwirbel-Körbchen 2 (Interbody Cage) einzusetzen.

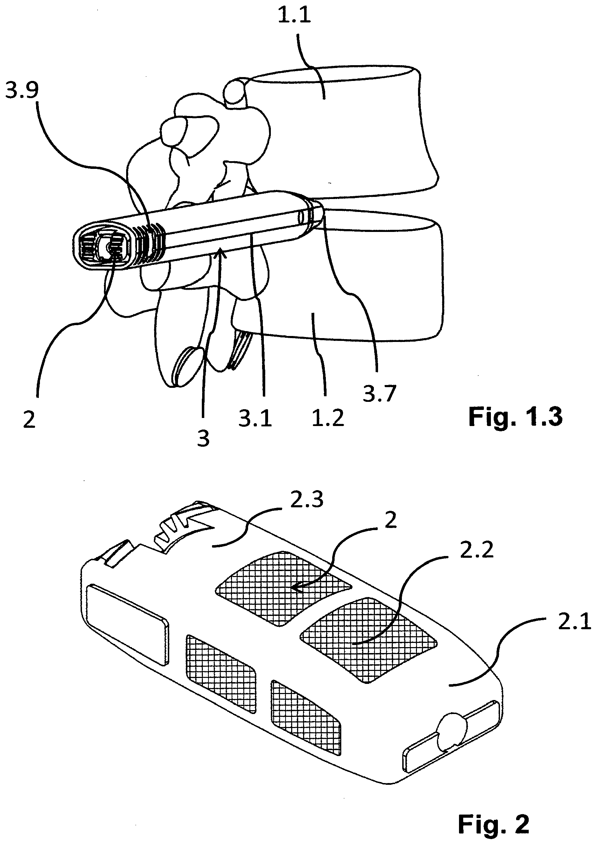

Ein Zwischenwirbel-Körbchen 2 kann unterschiedliche Konturen aufweisen. Als geeignet hat sich ein Körbchen 2 herausgestellt, wie es beispielhaft in der Fig. 2 perspektivisch dargestellt ist. Ein solches Körbchen weist einen stabilen äußeren Rahmen 2.1 und eine innere poröse, gitter- oder netzförmige Struktur 2.2 auf, die es dem Knochen der benachbarten Wirbel 1.1, 1.2 erlaubt, in diese Struktur einzuwachsen, um so eine feste Verbindung zu schaffen.

Wie ersichtlich, verjüngt sich das Körbchen in Längsrichtung zu seinem distalen Ende hin. Die obere Fläche 2.3 ist über die Breite hin konvex mit einem relativ großen Krümmungsradius gebogen. Dies ermöglicht eine Anpassung der Relativstellung der Wirbel über das Körbchen 2 hin. Demgegenüber ist die untere Fläche des Körbchens die (in Fig. 2 nicht sichtbare) untere Fläche des Körbchens, ebenso wie die Seitenwände über ihre Breite bzw. Höhe hin nicht bogenförmig, sondern zumindest nahezu eben ausgebildet.

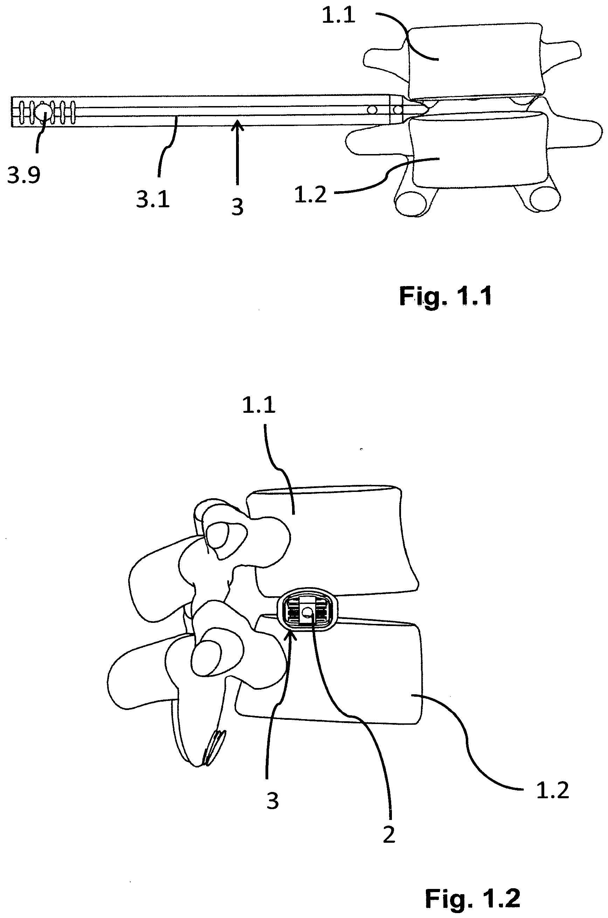

Ein solches Zwischenwirbel-Körbchen 2 wird, wie dies der Fig. 1.1 bis 1.3 entnehmbar ist, durch eine Führungshülse 3 in den Zwischenwirbelbereich bzw. das ehemalige Bandscheibenfach eingeführt.

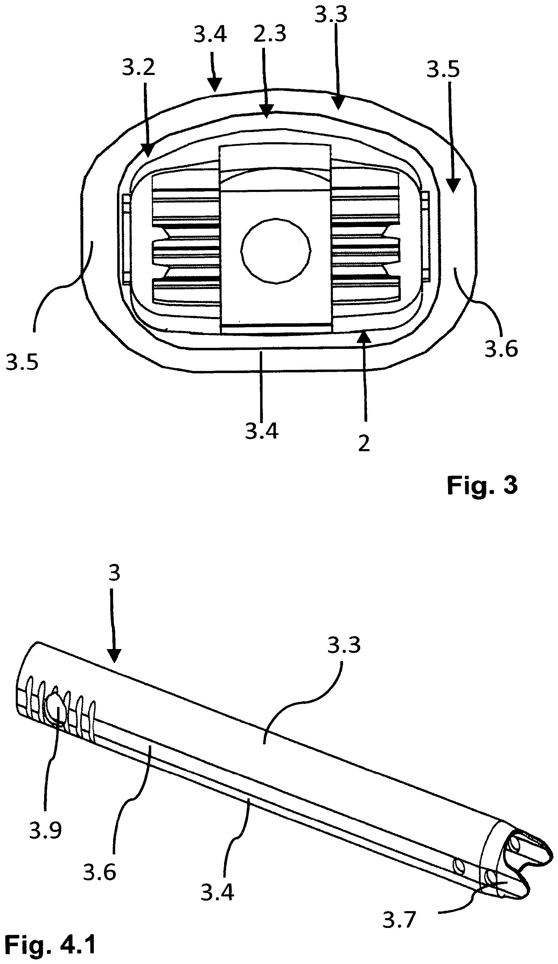

Wie insbesondere auch den Figuren 3 bis 4.2 zu entnehmen ist, weist die Führungshülse 3 weist über ihre gesamte Länge hin einen nicht-zylindrischen Mantel 3.1 und ein (inneres) Lumen 3.2 mit einem nicht-zylindrischen konstanten Lumenquerschnitt auf. Die Querschnittskontur des Lumens 3.2 der Führungshülse 3 ist an die maximale Querschnittskontur des einzuführenden Zwischenwirbel-Körbchens 2 (Fig. 2), also dessen Querschnittskontur kurz vor dem proximalen Ende des Körbchens 2 mit hinreichender Toleranz angepasst.

Der Mantel 3.1 bzw. die Wandung der Führungshülse 3 weist im Wesentlichen einander gegenüberliegende obere und untere Breitseiten 3.3, 3.4 sowie diese verbindenden Schmalseiten 3.5, 3.6 auf, im vorliegenden Ausführungsbeispiel zwei einander gegenüberliegende Schmalseiten 3.5, 3.6.

Die untere Breitseite 3.4 ist im Wesentlichen, ebenso wie die Schmalseiten 3.5, 3.6, auf der Innenseite zum Lumen hin eben ausgebildet, wobei der Übergang zwischen der Breitseite 3.4 und den Schmalseiten 3.5, 3.6 nicht scharfwinklig, sondern abgerundet ist - der Krümmungsradius des Übergangs zwischen unterer Breitseite 3.4 und Schmalseiten 3.5, 3.6 beträgt hier 4 mm.

Die obere Breitseite 3.3 ist über die Breite der Führungshülse 3 hin nach außen konvex gebogen ausgebildet (also nicht eben). Der Krümmungsradius der oberen Breitseite beträgt hier 12 mm. Die Hülse weist an ihrer proximalen Au-ßenseite Quernuten 3.8 auf sowie diametral gegenüberliegende Wandungsdurchbrüche 3.9 auf. Durch die Ausgestaltung wird die Griffigkeit für Finger des Operateurs bzw. Werkzeuge verbessert.

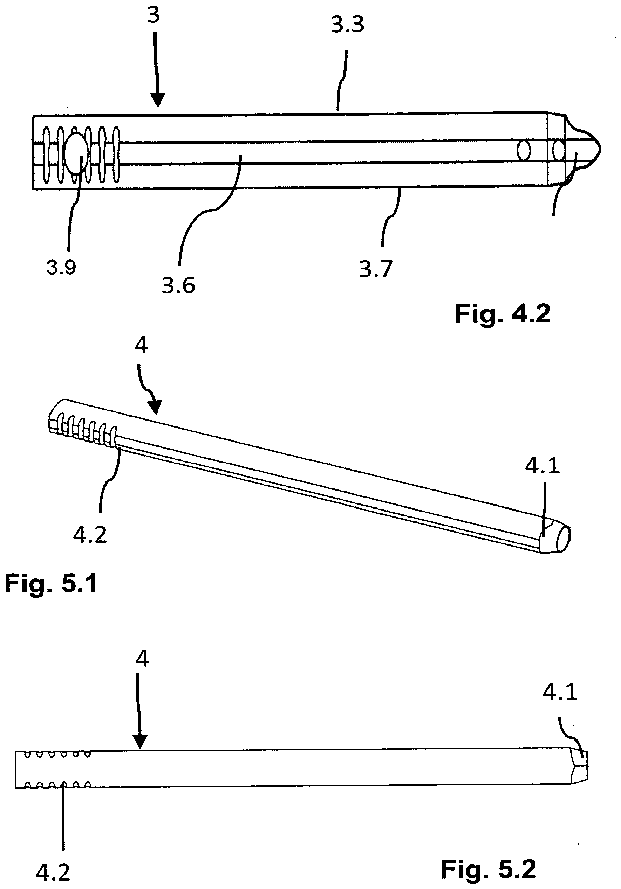

Wie ebenfalls insbesondere aus der Fig. 3 ersichtlich ist, ist die Wandungsstärke der oberen und unteren Breitseite 3.3, 3.4 geringer als die Wandungsstärke der Schmalseiten 3.5, 3.6. Während typischerweise im dargestellten Ausführungsbeispiel die Wandstärke der oberen und unteren Breitseite 3.3, 3.4 ca. 1 mm beträgt, beträgt die Wandungsstärke der Schmalseiten 3.5, 3.6 ca. 1,5 mm (die Darstellung der Fig. 3 ist eine vergrößerte Darstellung). Am distalen Ende der Schmalseiten 3.5, 3.6 sind - distale - Vorsprünge 3.7 ausgebildet (Fig. 1, 4.1 bis 4.2). Diese dienen zum einen zur festen Verankerung der Führungshülse zwischen den beiden Wirbeln 1.1 und 1.2 und zum anderen auch zur Aufweitung des Zwischenraums, damit das Körbchen 2 zwischen die beiden Wirbel 1.1, 1.2 eingeführt werden kann. Da die Vorsprünge 3.7 eine Fortsetzung der Schmalseiten 3.5, 3.6 bilden, wird durch die größere Wandungsstärke der Schmalseiten 3.5, 3.6 auf den Vorsprüngen 3.7 eine höhere Stabilität verliehen, so dass diese ihre Aufgabe einer sicheren Verankerung der Führungshülse 3 und einer Aufweitung des Zwischenraums zwischen den Wirbeln 1.1, 1.3 sicher nachkommen können.

Zwischenwirbel-Körbchen 2 und Führungshülse 3 bilden gemeinsam ein Set für Wirbelsäuleneingriffe. Ihre Querschnittskontur, die äußere des Körbchens 2 und die des Lumens der Führungshülse 3, sind aneinander angepasst.

Die Fig. 5.'1 und 5.2 zeigen einen Dilator 4, der unmittelbar innerhalb der Führungshülse 3 zu liegen kommt, wenn diese über ihn geschoben wird. Die Außenkontur des unmittelbar innerhalb der Führungshülse 3 befindlichen Dilators 4 ist demgemäß an das Lumen der Führungshülse 3 mit geringen Toleranzen angepasst. Das heißt, die untere äußere Breitseite des Dilators ist ebenso wie die äußeren Schmalseiten desselben weitgehend eben ausgebildet, während die obere äußere Breitseite nach außen hin konvex gebogen ist. Die äußere Querschnittskontur des Dilators 4 ist über den größten Teil seiner Länge konstant; lediglich das distale Ende 4.1 des Dilators 4 verjüngt sich konusförmig, wie das bei derartigen Dilatoren üblich ist.

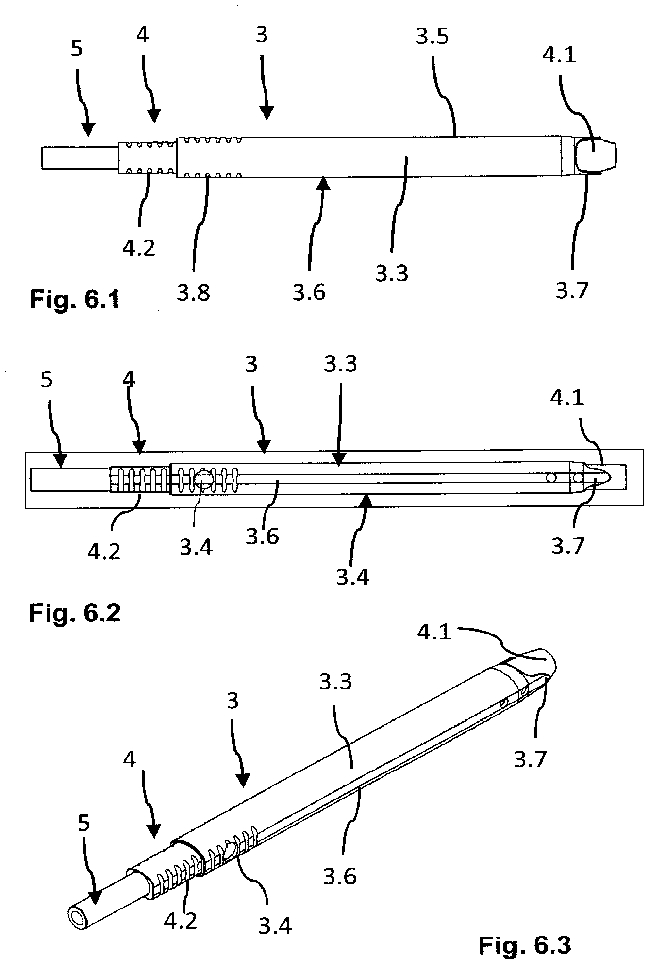

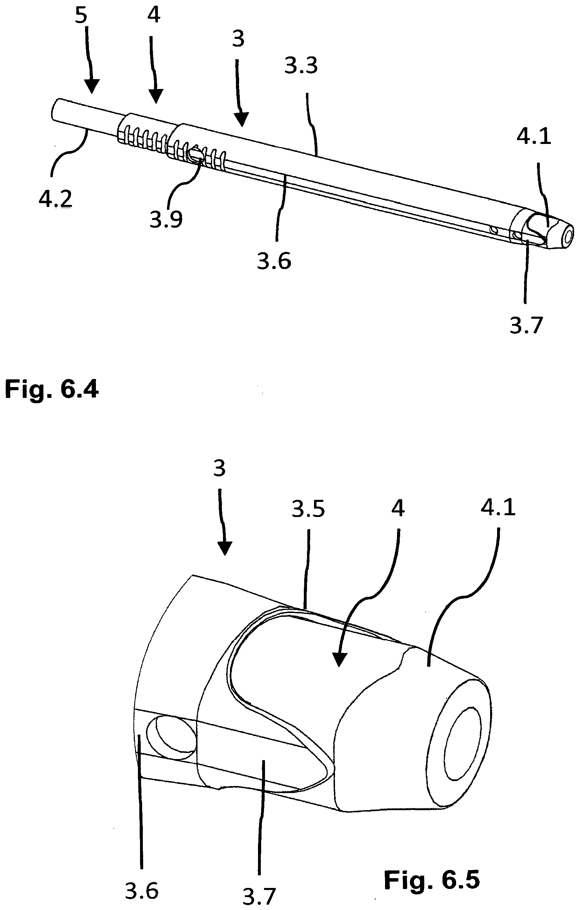

Das Innenlumen des Dilators 4 ist, wie in fig. 5.1 insbesondere am rechten - distalen - Ende des Dilators 4 in der, oder auch am linken - proximalen - Ende in der Fig. 6.3 zu sehen ist, zylinderförmig, also die proximale und distale Ein- bzw. Auslassöffnung sind kreisförmig. Auch der Dilator 4 weist an seiner proximalen Außenseite Quernuten 4.2 auf.

Die Fig. 6.1 bis 6.5 zeigen ein (Einführungs-)Set für einen Wirbelsäuleneingriff mit einer Führungshülse, wie sie vorstehend beschrieben wurde und zwei dargestellten Dilatoren den vorstehend insbesondere in Bezug auf die Fig. 5.1 und 5.2 beschriebenen Dilator 4 und einen weiteren Dilator 5, der innerhalb des Dilators 4 zu liegen kommt bzw. auf den der Dilator 4 aufgeschoben wird.

Der weitere in den Fig. 6.1 bis 6.5 dargestellte - weiter innenliegende - Dilator 5 hat entsprechend der zylindrischen Innenkontur des Dilators 4 eine zylinderförmige Außenkontur - und ebenfalls auch eine zylinderförmige Innenkontur -, wie dies bei derartigen Dilatoren üblich ist. Die beiden Einzelsets aus den Teilen 2, 3 einerseits und 3, 4, 5 andererseits bilden ein gesamtes Operationsset.

Der Zugang zum Zwischenraum (Bandscheibenfach) zwischen zwei Wirbeln 1.1, 1.2 mittels der erfindungsgemäßen Vorrichtung ist sowohl posterior-lateral P als auch anterior-lateral A möglich und zwar vorzugsweise mit einem Zugangswinkel von jeweils 30° bis 60°, vorzugsweise 40° bis 50° jeweils zur - mittleren - Sagittalebene S bzw. in diese liegende - horizontale - Sagittalachse jeweils oben von seitlich hinten bzw. vorne, wie dies in Fig. 7 dargestellt ist. Der posterior-laterale Zugang kann dabei vorzugsweise entlang der äußeren Seitenfläche des Dornfortsatzes 1.3 des Wirbels 1.1 erfolgen.

Durch die erfindungsgemäße nicht-zylinderförmige Ausgestaltung der erfindungsgemäßen Führungshülse wird erreicht, dass durch diese zwar einerseits ein Zwischenwirbel-Körbchen eingeführt werden kann, andererseits aber die Gewebebelastung eines Patienten im Einführbereich der Führungshülse 3 zwischen Hautoberfläche und Zwischenwirbelbereich, insbesondere in Richtung der Erstreckung der Wirbelsäule, möglichst wenig belastet wird, da durch die erfindungsgemäße Ausgestaltung der Führungshülse deren Höhe gegenüber herkömmlichen zylindrischen Führungshülsen (mit kreisförmigem Querschnitt) wesentlich reduziert werden kann.

## Patentansprüche

1. Vorrichtung für Wirbelsäuleneingriffe, mit mindestens einer Führungshülse, mit mindestens einem Dilator (4, 5), dessen Außenkontur der Innenkontur der Führungshülse entspricht, mit einem nicht-zylindrischen Mantel (3.1) der Führungshülse (3), **dadurch gekennzeichnet, dass** zwei einander gegenüberliegende Breitseiten (3.3, 3.4) eine größere Breite als die Breite dieser verbindender Schmalseiten (3.5, 3.6) haben, dass eine der Breitseiten (3.4) eben ist, dass eine andere Breitseite (3.3) konvex nach außen gebogen ist und dass der Krümmungsradius des Übergangs zwischen einer Breitseite (3.3, 3.4) und einer Schmalseite (3.5, 3.6) zwischen 3 und 4,5 mm liegt.

2. Vorrichtung nach Anspruch 1, **dadurch gekennzeichnet, dass** die konvex nach außen gebogene Breitseite (3.3) einen Krümmungsradius (R) von 11 bis 12,5 mm hat.

3. Vorrichtung nach Anspruch 1 oder 2, **dadurch gekennzeichnet, dass** die Schmalseiten (3.5, 3.6) distal die Breitseiten (3.3, 3.4) überragende Vorsprünge (3.7) aufweisen.

4. Vorrichtung nach Anspruch 3, **dadurch gekennzeichnet, dass** die Vorsprünge sich zu ihrem freien Ende hin verjüngen und am freien Ende abgerundet sind.

5. Vorrichtung nach einem der vorangehenden Ansprüche, **dadurch gekennzeichnet, dass** die Schmalseiten (3.5, 3.6) eine größere Stärke als die Breitseiten (3.3, 3.4) aufweisen, vorzugsweise die Stärke der Breitseiten (3.3, 3.4) 0,8 mm bis 1,2 mm und/oder die Stärke der Schmalseiten (3.5, 3.6) größer 1,2 mm bis 1,7 mm beträgt.

6. Vorrichtung nach einem der vorangehenden Ansprüche, **dadurch gekennzeichnet, dass** die Hülse (3) senkrecht zu ihrer Längsachse verlaufende Nuten (4.8) im proximalen Bereich ihrer Außenseite, insbesondere an ihrer Schmalseite der Hülse (3) aufweist.

7. Vorrichtung nach einem der vorangehenden Ansprüche, **dadurch gekennzeichnet, dass** der Dilator (5) senkrecht zu seiner Längsachse verlaufenden Nuten (5.2) im proximalen Bereich seiner Außenseite, insbesondere an seiner Schmalseite aufweist.

8. Vorrichtung nach einem der vorangehenden Ansprüche, **gekennzeichnet durch** im proximalen Nadelbereich diametral gegenüberliegenden Durchbrüche (3.9).

9. Vorrichtung nach einem der vorangehenden Ansprüche, **dadurch gekennzeichnet, dass** das Lumen des Dilators (4, 5) zylindrisch ausgebildet ist.

10. Hülse nach Anspruch 9, **dadurch gekennzeichnet, dass** die Hülse (3) senkrecht zu ihrer Längsachse verlaufende Nuten (4.8) im proximalen Bereich ihrer Außenseite, insbesondere an ihrer Schmalseite der Hülse (3) aufweist und/oder dass im proximalen Nadelbereich diametral gegenüberliegenden Durchbrüche (3.9) vorgesehen sind.

## Claims

1. Device for spine surgery, with at least one guide sleeve, with at least one dilator (4, 5), whose outer contour corresponds to the inner contour of the guide sleeve, with a non-cylindrical jacket (3.1) of the guide sleeve (3), **characterized in that** two mutually opposite broad sides (3.3, 3.4) have a breadth greater than the breadth of narrow sides (3.5, 3.6) connecting these, that one of the broad sides (3.4) is flat, that another broad side (3.3) is bent convexly outwards and that the radius of curvature of the transition between a broad side (3.3, 3.4) and a narrow side (3.5, 3.6) is between 3 mm and 4.5 mm.

2. Device in accordance with claim 1, **characterized in that** the broad side (3.3) which is convexly bent outwards has a radius of curvature (R) of 11 mm to 12.5 mm.

3. Device in accordance with claim 1 or 2, **characterized in that** the narrow sides (3.5, 3.6) have distal projections (3.7) projecting over the broad sides (3.3, 3.4).

4. Device in accordance with claim 3, **characterized in that** the projections are tapered towards their free end and are round at their free end.

5. Device in accordance with one of the above claims, **characterized in that** the narrow sides (3.5, 3.6) have a greater thickness than the broad sides (3.3, 3.4), the thickness of the broad sides (3.3, 3.4) preferably being 0.8 mm to 1.2 mm and/or the thickness of the narrow sides (3.5, 3.6) being greater than 1.2 mm to 1.7 mm.

6. Device in accordance with one of the above claims, **characterized in that** the sleeve (3) has grooves (4.8) extending at right angles to its longitudinal axis in the proximal area of its outer side, especially on its narrow side of the sleeve (3).

7. Device in accordance with one of the above claims, **characterized in that** the dilator (5) has grooves (5.2) extending at right angles to its longitudinal axis in the proximal area of its outer side, especially on its narrow side.

8. Device in accordance with one of the above claims, **characterized by** openings (3.9) located diametrically opposite in the proximal area of the needle.

9. Device in accordance with one of the above claims, **characterized in that** the lumen of the dilator (4, 5) has a cylindrical configuration.

10. Device in accordance with claim 9, **characterized in that** the sleeve (3) has grooves (4.8) extending at right angles to its longitudinal axis in the proximal area of its outer side, especially on the narrow side of the sleeve (3) and/or that openings (3.9) are provided which are located diametrically opposite in the proximal area of the needle.

## Revendications

1. Dispositif pour des interventions sur la colonne vertébrale, avec au moins une douille de guidage, avec au moins un dilatateur (4, 5) dont le contour extérieur correspond au contour intérieur de la douille de guidage, avec une enveloppe (3.1) non cylindrique de la douille de guidage (3), **caractérisé en ce que** deux côtés larges (3.3, 3.4) opposés l'un par rapport à l'autre ont une largeur supérieure à la largeur de ces côtés étroits (3.5, 3.6) les reliant, qu'un des côtés larges (3.4) est lisse, qu'un autre côté large (3.3) est plié vers l'extérieur de façon convexe et que le rayon de courbure de la transition entre un côté large (3.3, 3.4) et un côté étroit (3.5, 3.6) se situe entre 3 et 4,5 mm.

2. Dispositif selon la revendication 1, **caractérisé en ce que** le côté large (3.3) incurvé vers l'extérieur de façon convexe a un rayon de courbure (R) de 11 à 12,5 mm.

3. Dispositif selon la revendication 1 ou 2, **caractérisé en ce que** les côtés étroits (3.5, 3.6) comportent des saillies (3.7) saillant distalement des côtés larges (3.3, 3.4).

4. Dispositif selon la revendication 3, **caractérisé en ce que** les saillies se rétrécissent en direction de leur extrémité libre et sont arrondies au niveau de leur extrémité libre.

5. Dispositif selon l'une quelconque des revendications précédentes, **caractérisé en ce que** les côtés étroits (3.5, 3.6) présentent une épaisseur plus importante que les côtés larges (3.3, 3.4), l'épaisseur des côtés larges (3.3, 3.4) étant de préférence de 0,8 mm à 1,2 mm et/ou l'épaisseur des côtés étroits (3.5, 3.6) étant supérieure de 1,2 mm à 1,7 mm.

6. Dispositif selon l'une quelconque des revendications précédentes, **caractérisé en ce que** la douille (3) comporte des rainures (4.8) s'étendant perpendiculairement à son axe longitudinal dans la région proximale de son côté extérieur, notamment au niveau du côté étroit de la douille (3).

7. Dispositif selon l'une quelconque des revendications précédentes, **caractérisé en ce que** le dilatateur (5) comporte des rainures (5.2) s'étendant perpendiculairement à son axe longitudinal dans la région proximale de son côté extérieur, notamment au niveau de son côté étroit.

8. Dispositif selon l'une quelconque des revendications précédentes, **caractérisé par** des passages traversants (3.9) opposés de façon diamétralement opposée dans la zone d'aiguille proximale.

9. Dispositif selon l'une quelconque des revendications précédentes, **caractérisé en ce que** le lumen du dilatateur (4, 5) est réalisé de façon cylindrique.

10. Douille selon la revendication 9, **caractérisée en ce que** la douille (3) comporte des rainures (4.8) s'étendant perpendiculairement à son axe longitudinal dans la zone proximale de son côté extérieur, notamment au niveau du côté étroit de sa douille (3) et/ou que des passages traversants (3.9) sont prévus de façon diamétralement opposée dans la zone d'aiguille proximale.
